# EUROPEAN PATENT SPECIFICATION

(11) **EP 2 450 457 B1**
(45) Date of publication and mention of the grant of the patent: **16.08.2017**
(21) Application number: 11008830.9
(22) Date of filing: 07.11.2011
(51) Int. Cl.: C12Q 1/68, G01N 33/574

(54) **Method of analyzing genetically abnormals cells**
Verfahren zum Analysieren genetisch abweichender Zellen
Procédé d'analyse de cellules anormales génétiquement

(30) Priority: 09.11.2010 JP 2010251107
(43) Date of publication of application: 09.05.2012
(73) Proprietor: Olympus Corporation, Tokyo 192-8507 (JP); Japanese Foundation For Cancer Research, Koto-ku Tokyo (JP)
(72) Inventor: Mishima, Yuji, Tokyo (JP); Hatake, Kiyohiko, Tokyo (JP); Abe, Takashi, Tokyo 192-8507 (JP)
(74) Representative: Schicker, Silvia

(56) References cited:
- WO-A1-00/20641
- WO-A2-01/20044
- US-A1- 2009 081 688
- US-A1- 2010 120 027
- WÜLFING PIA ET AL: "HER2-positive circulating tumor cells indicate poor clinical outcome in stage I to III breast cancer patients.", CLINICAL CANCER RESEARCH : AN OFFICIAL JOURNAL OF THE AMERICAN ASSOCIATION FOR CANCER RESEARCH 15 MAR 2006 LNKD- PUBMED:16551854, vol. 12, no. 6, 15 March 2006 (2006-03-15) , pages 1715-1720, XP002671702, ISSN: 1078-0432
- MUNZONE ELISABETTA ET AL: "Changes of HER2 status in circulating tumor cells compared with the primary tumor during treatment for advanced breast cancer.", CLINICAL BREAST CANCER 1 OCT 2010 LNKD- PUBMED:20920984, vol. 10, no. 5, 1 October 2010 (2010-10-01), pages 392-397, XP002671703, ISSN: 1938-0666
- FLORES L M ET AL: "Improving the yield of circulating tumour cells facilitates molecular characterisation and recognition of discordant HER2 amplification in breast cancer", BRITISH JOURNAL OF CANCER, vol. 102, no. 10, May 2010 (2010-05), pages 1495-1502, XP002671705,

## Description

### BACKGROUND OF THE INVENTION

### Field of the Invention

The present invention relates to a method for detecting and analyzing cells having gene abnormality using fluorescence in situ hybridization (hereinafter abbreviated as FISH).

### Description of the Related Art

Recently, molecularly-targeted drugs have intensively been developed. In particular, it can be expected that side effects are reduced as compared with conventional anticancer agents by using molecularly-targeted drugs, which act on a specific molecule involved in the onset and growth of cancer, in a cancer treatment. However, since molecularly-targeted drugs act on a specific molecule in vivo, sufficient therapeutic effects by medication sometimes cannot be expected depending on the genotype of the patient. Therefore, analysis of genetic mutation becomes more and more important for prediction of therapeutic effects of molecularly-targeted drugs.

For example, in various cancers such as breast cancer, ovarian cancer and stomach cancer, Her-2 gene amplification is carried out and a HER-2 protein is excessively expressed. It is said that breast cancer often has a poor prognosis when the HER-2 protein is excessively expressed. Also, a HER-2-targeting antibody drug (a humanized monoclonal antibody against HER-2) is effective against such HER-2-positive breast cancer, while therapeutic effects cannot be expected against HER-2-negative breast cancer in which excessive expression of the HER-2 protein is not recognized. Therefore, analysis of the presence or absence of Her-2 gene amplification is important so as to predict the presence or absence of adaptation of the HER-2-targeting antibody drug, a clinical course and the like.

In general, Her-2 gene amplification has been analyzed by FISH. Specifically, a nucleic acid probe (Her-2 probe) which binds to DNA of the Her-2 gene and a nucleic acid probe (CEP17 probe) which binds to a centromere DNA on chromosome 17 (CEP17) are labeled with different fluorescent substances, respectively, and the FISH is carried out using these two fluorescence-labeled nucleic acid probes. When the ratio of the total Her-2 signal count to the total CEP17 signal count (Her-2/CEP17 ratio) is a given value (for example, 2.0) or more, Her-2 gene amplification is positive (Her-2 gene amplification is carried out). When the ratio is less than the given value, it is determined that Her-2 gene amplification is negative.

Tumor cells systemically circulate and metastasize along the flow of blood and lymphocytes. Therefore, it is possible to test progression and metastatic property of tumor and to predict prognosis of tumor by detecting and analyzing tumor cells existing in the blood (peripheral circulatory tumor cells; CTCs). However, a large amount of leukocytes exist in blood, and a proportion of CTCs existing in blood is small. Moreover, it is difficult to determine whether cells are CTCs or not based on their morphology since a tissue structure of CTCs is not preserved unlike a tissue section or the like. Therefore, tumor cells are usually analyzed by separating and recovering epithelial cells or tumor cells from blood cells utilizing an antibody against a molecule specifically existing on the cell surface of epithelial cells or tumor cells, and then carrying out gene analysis, expression analysis or the like to detect the presence or absence of gene abnormality.

In a CTC analysis of breast cancer, for example, a method for analyzing whether cells are Her-2 gene amplification-positive cells or not is carried out by recovering EpCAM-positive cells from mononuclear cells in the blood using an antibody against EpCAM which is a cell surface antigen of epithelial cells, and carrying out FISH on the recovered EpCAM-positive cells using a Her-2 probe and a CEP17 probe to determine a Her-2/CEP17 ratio (see, for example, Non Patent Document 1).

The method for analyzing CTCs includes, in addition to the above method, a method in which floating cells having a cell surface antigen fluorescently stained are subjected to a fluorescence in situ hybridization method, and then nuclei are observed by focusing on the nuclei of the cells using a fluorescent microscope (see, for example, Patent Document 1). There is also a method in which an antibody, which specifically binds to biogenic substances other than a chromosome such as a cell surface antigen, and also has a reaction complex that cannot be detected with a fluorescent microscope, is bound to cells and the cells are subjected to FISH on the cells, and then a fluorescence-labeled substance, which reacts with the reaction complex, is bound to the cells (see, for example, Patent Document 2). A single cell is subjected to fluorescent immunostaining and FISH, and thus making it possible to reduce the requisite amount of a single specimen as compared with a case in which both are separately carried out.

### PRIOR ART DOCUMENTS

### PATENT DOCUMENTS

[Patent Document 1] Japanese Laid-Open Patent Application No. 2007-178193
[Patent Document 2] Japanese Laid-Open Patent Application No. 2009-530621

### NON PATENT DOCUMENTS

[Non Patent Document 1] Flores et al., British Journal of Cancer, 2010, Vol. 102, No. 10, p.1495-1502

### DISCLOSURE OF THE INVENTION

### PROBLEMS TO BE SOLVED BY THE INVENTION

A method for analyzing recovered cells using a molecule specifically existing on the cell surface of epithelial cells or tumor cells as a marker, like a method carried out in a conventional analysis of CTCs, has a problem in that CTCs, which do not express a molecule serving as a marker, are not included in the detection objects.

For example, in the above method for analyzing CTCs of breast cancer, it is impossible to detect EpCAM-negative CTCs.

In order to solve the above problem, a method is proposed in which a molecule specifically existing on the blood cell surface is conversely used as a marker and cells expressing the marker from a specimen, and then analysis of the remaining cells is carried out. Whereby, it is possible to reduce a risk that CTCs negative in a particular marker are excluded from the detection objects. However, it is usually impossible to completely remove blood cells, and still many blood cells are mixed in recovered cells. Thus, it is very difficult to detect CTCs existing only in a trace amount by such a method.

On the other hand, in a method in which all cells in a specimen are subjected to fluorescent immunostaining against a cell surface antigen and FISH, like the method described in Patent Document 1, all CTCs contained in the specimen can be the analysis objects without separating cells from the specimen. Since a gene of cells in which the presence of a particular cell surface antigen is confirmed by fluorescent immunostaining can be analyzed by FISH, an improvement in analytical accuracy can be expected. Particularly, in the method described in Patent Document 1, it is possible to detect fluorescence signals of FISH without blocking the signals by a fluorescent dye used for fluorescent immunostaining by focusing on the nuclei of cells when observing cells after carrying out fluorescent immunostaining and FISH under a fluorescent microscope.

However, in the method described in Patent Document 1, the observer observes each cell by focusing on a nucleus of the cell under a fluorescent microscope. In other words, the observer must manually manipulate the fluorescent microscope, and much labor is required as the number of cells for observation increases.

Also, when analysis is carried out based on a two-dimensional image obtained by focusing on a particular position in cells, for example, multiple signals which have different heights in the focus direction of a microscope but exists at the same position on a focal plane (plane of two-dimensional image), i.e., multiple signals existing at a three-dimensionally overlapping position, overlap with one another and are detected as one bright spot, thereby sometimes causing a reduction in detection accuracy. Particularly, in epi-observation, fluorescent immunostaining against a cell surface antigen and fluorescence signals of FISH provide an overlapped image. Moreover, when nuclear staining is simultaneously used, an image is provided in which signals of the nuclear staining also overlap in addition to the above signals. Accordingly, it is very difficult to carry out accurate analysis from a 2D image. On the other hand, when a confocal optical system is used, observation is carried out on a confocal plane, and therefore, there is no overlap in an image and a very clear image is obtained as compared with the case of epi-observation. However, in the method described in Patent Document 1, focus is placed on a position at which a nucleus is most clearly observed, and it is determined that a bright spot clearly observed within the nucleus on the focal plane is a FISH signal and that a bright spot which is out of focus is a signal by fluorescent immunostaining. Since a sequence of operations leading from setup of the focal position to determination of signals are submitted to the observer, observer variation may occur.
Wülfing et al. (Clin Cancer Res. 12(6): 1715-1720 (2006) discloses a method for isolating Her-2positive circulating tumor cells (CTC) and subsequent analysis by immunocytochemistry.

WO 00/20641 discloses a method combining immunohistochemistry with FISH within the same section of a tissue sample.

### SUMMARY OF INVENTION

An object of the present invention is to provide a method for detecting and analyzing cells having gene abnormality in a highly accurate and simple manner using FISH.

The present inventors have diligently conducted experiments so as to achieve the above object and found that cells having gene abnormality can be detected in a highly accurate and simple manner even when only a trace amount of the cells exist in a specimen by carrying out immunostaining against a cell surface antigen and FISH to the same cell group, and carrying out analysis of fluorescence signals from the cell group using a three image analysis method. Thus, the present invention was achieved.

The present invention relates to following (1) to (8).
(1) a method of analyzing genetically abnormal cells, the method including the steps of:
   (a) preparing a cell sample containing eukaryotic cells, wherein the cell sample is blood or a mononuclear cell component separated from blood,
   (b) immunostaining of cells contained in the cell sample using one or more antibodies against a cell surface antigen of genetically abnormal cells after the step (a),
   (c) subjecting the cells contained in the cell sample to a permeation treatment after the step (b),
   (d) subjecting the cells contained in the cell sample to an immobilization treatment after the step (b),
   (e) conducting FISH (fluorescence in situ hybridization) to the cells contained in the cell sample using one or more nucleic acid probes specifically binding to DNA of a cancer gene after the step (d),
   (f) analyzing fluorescence signals from the nucleic acid probes in the cells contained in the cell sample using a three-dimensional image analysis method after the step (e), and
   (g) determining whether the cells contained in the cell sample are genetically abnormal cells or not based on the results of the step (b) and the results of the step (f) by analyzing results of the FISH on cells having bound the antibody of the step (b), further comprising the step of (i) immunostaining of cells contained in the cell sample using one or more antibodies which specifically bind to a molecule existing in the interior of cells likely to be genetically abnormal before the step (g) after the step (c).
(2) The method of analyzing genetically abnormal cells according to the above (1), wherein the permeation treatment is carried out by an enzyme reaction treatment with a proteolytic enzyme after an immersion treatment in a surfactant solution.
(3) The method of analyzing genetically abnormal cells according to the above (1) or (2), further including the step of:
   (h) removing cells binding to the antibody from the cell sample prepared in the step (a) using an antibody which specifically binds to a molecule existing on the cell surface of other cells than genetically abnormal cells which are analysis objects before the step (b), wherein
   the step (b) is the step of subjecting cells remaining after removal of a portion of cells by the step (h) to an antigen-antibody reaction.
(4) The method of analyzing genetically abnormal cells according to any one of the above 1 to 3, wherein the cell sample is a body fluid, a sample containing cells separated from a body fluid, or a sample containing cells separated from a piece of tissue collected from the living body.
(5) The method of analyzing genetically abnormal cells according to any one of the above (1) to (4), wherein the nucleic acid probe is a nucleic acid probe specifically binding to DNA of the Her-2 gene, and a nucleic acid probe specifically binding to DNA of the CEP17 gene.
(6) The method of analyzing genetically abnormal cells according to any one of the above (3), wherein the antibody used in the step (h) is an antibody which specifically binds to a molecule specifically existing on the cell surface of leukocyte cells; and the nucleic acid probe is a nucleic acid probe specifically binding to DNA of the Her-2 gene, and a nucleic acid probe specifically binding to DNA of the CEP17 gene.
(7) The method of analyzing genetically abnormal cells according to the above (3), wherein
   the antibody used in the step (b) is an antibody which specifically binds to a molecule specifically existing on the cell surface of leukocyte cells;
   the antibody used in the step (h) is an antibody which specifically binds to a molecule specifically existing on the cell surface of leukocyte cells;
   the antibody used in the step (i) is an antibody which specifically binds to one or more proteins selected from the group consisting of Cytokeratin, CD45, HER-2, and EpCAM; and
   the nucleic acid probe is a nucleic acid probe specifically binding to DNA of the Her-2 gene, and a nucleic acid probe specifically binding to DNA of the CEP17 gene.
(8) The method of analyzing genetically abnormal cells according to any one of the above (2) to (7), wherein the surfactant solution is triton X-100; and the proteolytic enzyme is pepsin.

According to the method for analyzing genetically abnormal cells of the present invention, it is possible to detect and analyze cells having gene abnormality with a high accuracy and simple manner. In particular, the method for analyzing genetically abnormal cells of the present invention is suitable for analyzing genetically abnormal cells such as CTCs which exist in a specimen only in a trace amount.

### BRIEF DESCRIPTION OF THE DRAWINGS

Fig. 1 shows two-dimensional images prepared from three-dimensional images reconstructed with respect to one visual field containing Cytokeratin-positive cells in Example 1.
Fig. 2 shows two-dimensional images prepared from three-dimensional images reconstructed with respect to one visual field containing Cytokeratin-positive cells in Example 1.
Fig. 3 shows two-dimensional images prepared from three-dimensional image reconstructed with respect to a certain visual field in Example 1.
Fig. 4 shows two-dimensional images prepared from three-dimensional images reconstructed with respect to one visual field containing Cytokeratin-positive cells in Example 2.
Fig. 5 shows two-dimensional images prepared from three-dimensional images reconstructed with respect to one visual field containing Cytokeratin-positive cells in Example 3.
Fig. 6 shows two-dimensional images prepared from one visual field containing Cytokeratin-positive cells in Reference Example 1.

### DETAILED DESCRIPTION OF THE INVENTION

The method for analyzing genetically abnormal cells of the present invention is characterized in that the same cells are subjected to immunostaining against a cell surface antigen and FISH. Since fluorescence signals of FISH are smaller than conventional immunostaining, it is often difficult to detect genetically abnormal cells among many cells only based on fluorescence signals of FISH. In the present invention, it is possible to detect genetically abnormal cells efficiently by carrying out immunostaining against a cell surface antigen, discriminating between cells that are likely to be genetically abnormal cells and cells not having a particular gene abnormality which is the analysis object among many cells contained in a cell sample, and preferentially analyzing fluorescence signals of FISH on cells of a cell group which possibly contain genetically abnormal cells, as defined in the claims. Also, immunostaining against a cell surface antigen is carried out to discriminate cell groups, and fluorescent immunostaining and FISH signals from cells after FISH are analyzed using a three-dimensional image analysis method, thereby making it possible to clearly and simply distinguish between an immunostaining image of a cell surface and fluorescence signals of FISH within a cell nucleus.

The gene abnormality which is the object of analysis in the method for analyzing genetically abnormal cells of the present invention may be a congenital abnormality or an acquired abnormality such as somatic mutation.

There is no particular limitation on the genetically abnormal cells which are objects of analysis in the method for analyzing genetically abnormal cells of the present invention as long as they are cells having gene abnormality, and are preferably cells having gene abnormality which is frequently observed in tumor cells. Examples of the genetically abnormal cells include cells having Her-2 gene amplification, cells having a Philadelphia chromosome and the like. The Philadelphia chromosome is a specific gene abnormality observed in chronic myeloid leukemia or the like.

The method for analyzing genetically abnormal cells of the present invention includes the steps of:
(a) preparing a cell sample containing eukaryotic cells, wherein the cell sample is blood or a mononuclear cell component separated from blood,
(b) immunostaining of cells contained in the cell using one or more antibodies against a cell surface antigen of genetically abnormal cells (or non-genetically abnormal cells)after the step (a),
(c) subjecting cells contained in the cell sample to a permeation treatment after the step (b),
(d) subjecting cells contained in the cell sample to an immobilization treatment after the step (b),
(e) conducting FISH (fluorescence in situ hybridization) to the cells contained in the cell sample using one or more nucleic acid probes specifically binding to DNA of a cancer gene after the step (d),
(f) analyzing fluorescence signals from the nucleic acid probes in cells contained in the cell sample using a three-dimensional image analysis method after the step (e), and
(g) determining whether cells contained in the cell sample are genetically abnormal cells or not from the results of the step (b) and the results of the step (f) by analyzing results of the FISH on cells likely to be genetically abnormal (or non-genetically abnormal) before the step (g) after the step (c), as defined in the claims.

Each step will be described below.

First, as the step (a), a cell sample containing eukaryotic cells is prepared, wherein the cell sample is blood or a mononuclear cell component separated from blood. Examples of the cell sample include a biological sample, cells separated from a biological sample, a culture of cells separated from a biological sample, a cultured cell and the like.

The cell sample used in the present invention is blood, or cells separated therefrom, and particularly preferably a mononuclear cell component. In this case, the preparation of a mononuclear cell component can be carried out by a conventional method such as a centrifugation method.

The cell sample may be provided for a next step (b) as it is, or may be used in the step (b) after removing at least a portion of cells having no specific gene abnormality which is the analysis object (non-genetically abnormal cells) such as normal cells contained in the cell sample. It is possible to increase the proportion of genetically abnormal cells in a cell sample subjected to the subsequent step and to increase detection sensitivity of genetically abnormal cells by removing non-genetically abnormal cells from a cell sample.

Specifically, removal of non-genetically abnormal cells can be carried out using an antibody which specifically binds to a molecule existing on the cell surface of non-genetically abnormal cells which are objects of analysis. It is possible to remove at least a portion of non-genetically abnormal cells which bind to the antibody from the cell sample by bringing a cell sample into contact with a carrier having an antibody bound and then separating the cell sample from the carrier.

When the cell sample is blood or a mononuclear cell component and genetically abnormal cells which are objects of analysis are CTCs, it is preferable to remove a portion or all of blood cell components such as leukocytes from the cell sample. Specifically, an antibody-bound carrier, which is prepared by binding an antibody of which an antigen is a molecule existing on the cell surface of leukocytes, platelets, red blood cells, or endothelial cells to a carrier such as magnetic beads or an agarose gel, is added to and mixed with blood or a mononuclear cell component. Subsequently, a liquid component after removing the carrier is used in the following step (b). Examples of antigens existing on the cell surface include CD45 existing on the surface of leukocytes, CD61 existing on the surface of platelets, CD235a existing on the surface of red blood cells, CD31 existing on the surface of endothelial cells and the like.

Examples of the technique for increasing the proportion of genetically abnormal cells in a cell sample include a method in which genetically abnormal cells are separated and recovered from a cell sample using an antibody against a surface antigen of genetically abnormal cells. When a particular cell group is separated and recovered using an antibody against a cell surface antigen. However, it is impossible to recover and analyze genetically abnormal cells having no surface antigen binding to an antibody used. Accordingly, it is preferred in the present invention that non-genetically abnormal cells are separated and removed from a cell sample so as to increase the proportion of genetically abnormal cells.

Next, as the step (b), an antigen-antibody reaction is carried out on cells contained in the above cell sample using one or more antibodies which specifically bind to a molecule existing on the cell surface of eukaryotic cells. It is possible to distinguish between a cell group containing genetically abnormal cells and a cell group not containing genetically abnormal cells by the presence or absence of the antigen-antibody reaction. For example, genetically abnormal cells can be detected and analyzed efficiently by carrying out an antigen-antibody reaction using a labeled antibody against a cell surface antigen of genetically abnormal cells, and preferentially analyzing, in subsequent FISH, fluorescence signals of cell nuclei in cells stained by binding of the labeled antibody (immunostaining). Conversely, genetically abnormal cells can be detected and analyzed efficiently by carrying out an antigen-antibody reaction using an antibody against a cell surface antigen of non-genetically abnormal cells, and preferentially analyzing, in subsequent FISH, fluorescence signals of cell nuclei in cells not having a labeled antibody bound and thus not stained.

The antigen-antibody reaction in the step (b) is a reaction in which a cell surface antigen is bound to an antibody specifically binding to the cell surface antigen. Thus, in the case of a direct method for immunostaining methods in which an antibody specifically binding to a cell surface antigen is directly labeled, all steps of the immunostaining are included in the step (b). On the other hand, in the case of an indirect method in which an antibody specifically binding to a cell surface antigen is used as a primary antibody and a secondary antibody (its antigen is the primary antibody) or streptavidin is labeled, only a reaction step in which the primary antibody is bound to a cell surface antigen is included in the step (b). In case a cell surface antigen is immunostained by an indirect method, an antigen-antibody reaction of the primary antibody bound to a cell surface antigen with a labeled secondary antibody or streptavidin may be carried out at any point before the step (f). For example, the reaction may be carried out successively with the step (b), simultaneously with the step (c), or after the step (d). When immunostaining is subsequently carried out on a molecule within cells, the reaction may be carried out simultaneously with the immunostaining.

Detection of an antibody bound to a cell surface antigen may be carried out by a fluorescence labeling method in which the antibody is labeled with a fluorescent substance or a fluorescent semiconductor quantum dot, or by an enzyme labeling method which uses a peroxidase-labeled antibody or an alkaline phosphatase-labeled antibody. In the present invention, the detection is preferably carried out using a fluorescence labeling method because it has high detection sensitivity and allows concomitant FISH analysis.

In the present invention, an antigen-antibody reaction of a cell surface antigen with an antibody specifically binding to the cell surface antigen is carried out on cells before a permeation treatment. Thus, in the case of a direct method, the permeation treatment is carried out after completion of an immune reaction. On the other hand, in the case of an indirect method, the permeation treatment is not carried out before completion of an antigen-antibody reaction of a primary antibody with cells. By carrying out an antigen-antibody reaction of a cell surface antigen with an antibody while not damaging a cell membrane, only the surface of the cell membrane can be stained unlike the case of carrying out the reaction after the permeation treatment. In other words, there is no overlap with the fluorescence signals of FISH and the S/N of FISH is improved by staining only the cell surface.

Next, as the step (c), a permeation treatment is carried out on cells contained in a cell sample. A pore is formed in a cell membrane or a nuclear membrane so that a nucleic acid probe for FISH can permeate into a nucleus by the permeation treatment. Examples of the permeation treatment include a treatment of immersing cells in a surfactant solution, an enzyme reaction treatment with a proteolytic enzyme and the like. It is possible to use, as the surfactant, Triton X-100, Tween 20, NP-40, saponin, digitonin and the like. It is possible to use, as the proteolytic enzyme, conventional enzymes such as pepsin and Proteinase K.

In the present invention, it is preferable to carry out an enzyme reaction treatment with a proteolytic enzyme, as a permeation treatment, after an immersion treatment in a surfactant solution. When the objects of analysis are CTCs, for example, the permeation treatment can be carried out by immersing cells in a 0.1 to 0.5% Triton X-100 solution for 1 to 30 minutes, preferably in a 0.1 to 0.3% Triton X-100 solution for 10 to 20 minutes, and more preferably in a 0.2% Triton X-100 solution for 15 minutes at room temperature, and then carrying out a pepsin treatment at room temperature for 0.5 to 10 minutes, preferably 0.5 to 5 minutes, and more preferably 1 to 2 minutes. Alternatively, the permeation treatment can be carried out by subjecting cells to an enzyme reaction treatment with a proteolytic enzyme such as a pepsin treatment without carrying out an immersion treatment in a surfactant solution.

When an immersion treatment in a surfactant solution and an enzyme reaction treatment with a proteolytic enzyme are carried out as a permeation treatment, both the treatments may be completed after an antigen-antibody reaction of a cell surface antigen with an antibody and before FISH. Both the treatments may be carried out continuously or discontinuously. In the present invention, it is preferable to carry out firstly an immersion treatment in a surfactant solution, then an immobilization treatment described below, and then an enzyme reaction treatment with a proteolytic enzyme.

After the step (b), as the step (d), an immobilization treatment of cells contained in a cell sample after an antigen-antibody reaction is carried out. It is possible to suppress denaturation of cells and an antibody bound by the immobilization treatment. Examples of the solution for the immobilization treatment include formalin, paraformaldehyde, glutaraldehyde, methanol, ethanol, acetone and the like. Treatment conditions such as types and concentrations of immobilization treatment solutions used for the immobilization treatment and treatment time can be determined suitably by taking account of types of cells, methods for immunostaining and the like so that cells and antibodies can be immobilized stably in subsequent steps and morphology of cells does not change so much. In the present invention, formalin is preferably used as an immobilization treatment solution.

After the step (b) and before the step (e), a crosslink treatment may be carried out on cells contained in a cell sample. It is possible to keep the binding of a cell surface antigen with an antibody stable by carrying out the crosslink treatment. In this case, the crosslink treatment may be carried out before a permeation treatment in the step (c) or an immobilization treatment in the step (d), or may be carried our after the permeation treatment or the immobilization treatment.

Examples of crosslinkers used for the crosslink treatment include DSS (Disuccinimidyl suberate), BS3 (Bis-sulfosuccinimidyl suberate) and the like. Treatment conditions such as types and concentrations of crosslinkers used and treatment time can be determined suitably by taking into account of the types of cells, methods for immunostaining and the like so that cells can be crosslinked with an antibody used in an immune reaction and fluorescence signals of subsequent FISH are not reduced largely. When the analysis objects are CTCs in the present invention, preferably the crosslink treatment is not carried out or the BS3 treatment is carried out, and more preferably the crosslink treatment is not carried out.

After the permeation treatment, as the step (e), FISH is carried out on cells contained in a cell sample using one, or two or more nucleic acid probes. Specifically, cells are immersed in a solution containing fluorescence-labeled nucleic acid probes which can bind to DNA in a chromosome which is the analysis object. The "nucleic acid probe which can bind to DNA" means a nucleic acid probe which can hybridize with a DNA fragment.

Nucleic acid probes used in the present invention may be those contributing to discrimination between cells having gene abnormality and normal cells, for example, those which can bind to DNA in a region having gene abnormality which is the object of analysis. In the present invention, a nucleic acid probe which specifically binds to DNA of the cancer gene is used as a nucleic acid probe because tumor cells having gene abnormality can be analyzed.

In order to analyze the presence or absence of Her-2 gene amplification, for example, it is preferable to use a nucleic acid probe specifically binding to the Her-2 gene in combination with a nucleic acid probe specifically binding to DNA of CEP17. In this case, it can be determined that, when the Her-2/CEP17 ratio of a certain cell a given value or more, for example, 2.0 or more, preferably 2.2 or more, the cell is a genetically abnormal cell in which the Her-2 gene is abnormally amplified.

In order to analyze a Philadelphia chromosome, it is preferable to use a nucleic acid probe specifically binding to DNA of the c-abl gene in combination with a nucleic acid probe specifically binding to DNA of the bcr gene.

Next, as the step (f), fluorescence signals from the above nucleic acid probes in cells contained in the above cell sample are analyzed using a three-dimensional image analysis method. Thus, in the present invention, fluorescence signals in FISH are analyzed using a three-dimensional image analysis method. Specifically, in the three-dimensional image analysis method, two-dimensional images (tomographic images) of cross-sections of cells are shot and obtained sequentially by shifting focal length, and these images are combined to reconstruct a three-dimensional image. It is possible to detect labeled cells among many cells in a cell sample simply without extracting or recovering cells using the image analysis method. On the other hand, in a two-dimensional image analysis method, there is a possibility that multiple fluorescence signals overlapping in focus direction are misidentified as one fluorescence signal or a fluorescence signal by an immunostaining method. When signals are observed by a fluorescent microscope, it is necessary to observe cells by shifting focal length in order to avoid such a misidentification, and therefore, it is difficult to analyze many cells. To the contrary, by analyzing using a three-dimensional image analyzer, it is possible to analyze many cells at a time rapidly and simply with high accuracy.

Fluorescence signals in FISH can be detected and analyzed, for example, using a cell image analyzer which has a confocal optical system and allows fluorescence detection. Specifically, it is possible to use a confocal fluorescent microscope having an image capturing means such as a CCD camera, and the like. Also, when signals are analyzed by a two-dimensional image analysis method as described in Patent Document 1 or the like, FISH signals must be detected by observing cells as an observer gradually shifts focal length. Therefore, it is necessary for the observer to carry out observation by a microscope over a long period of time. To the contrary, in the present invention, it is possible to automatically construct a three-dimensional image in a single cell and then to analyze the resulting three-dimensional image on another day at another place. Also, it is easy to re-examine the image by preserving the three-dimensional image.

Finally, as the step (g), it is determined whether cells contained in the above cell sample are genetically abnormal cells or not, from the results of the antigen-antibody reaction of the step (b) and the results of the FISH of the step (f). It is determined whether the cells are genetically abnormal cells or not by analyzing the results of the FISH preferentially on cells determined as having a possibility of being genetically abnormal cells as a result of the antigen-antibody reaction.

For example, when a cell sample is blood or a mononuclear cell component and genetically abnormal cells which are analysis objects are CTCs, and in case an antigen-antibody reaction in the step (b) is carried out using an antibody which specifically binds to a molecule such as CD45 (cell surface antigen) specifically existing on the cell surface of leukocyte cells and FISH in the step (f) is carried out using a nucleic acid probe specifically binding to an amplification site observed in gene Her-2 gene amplification together with a nucleic acid probe specifically binding to DNA of the CEP17 gene, it can be determined that cells having a Her-2/CEP17 ratio of a given value or more, among cells to which an antibody does not bind in an antigen-antibody reaction in the step (b), are genetically abnormal cells.

After the permeation treatment of the step (d) and before the step (g), it is also possible to carry out immunostaining using one or more antibodies which specifically bind to a molecule existing in the interior of eukaryotic cells. In case a crosslink treatment is carried out in the present invention, for example, it is also possible to carry out immunostaining against a molecule within cells after the permeation treatment and then the crosslink treatment. The immunostaining can be carried out by a conventional method as in the case of an antigen-antibody reaction in the step (b). Intracellular molecules which serve as an antigen may be those existing within genetically abnormal cells, or may be those existing within non-genetically abnormal cells. Also, antibodies used in this case may be those contributing to discrimination between cells having gene abnormality and normal cells, or may be those used for further analyzing cells which are proved to be genetically abnormal cells.

In addition, cell nuclei may be stained after a permeation treatment of the step (d) and before the step (g) in the present invention. Each cell can be recognized more easily in an image analysis method by staining cell nuclei. In this case, cell nuclei can be stained by a conventional method suitably using a conventional nuclear staining agent such as DAPI (4',6-diamino-2-phenylindole), PI (propidium iodide), Hoechst and the like.

For example, when a cell sample is blood or a mononuclear cell component and genetically abnormal cells which are analysis objects are CTCs, it is possible to discriminate more clearly between blood cells and epithelial cells containing CTCs, together with an antigen-antibody reaction on the cell membrane surface in the step (b), using an antibody against a molecule mainly existing on leukocytes such as CD45, an antibody against a molecule mainly existing on the cell surface of epithelial cells such as EpCAM, an antibody against a molecule mainly existing in the interior of epithelial cells such as Cytokeratin, and the like. It is possible to detect genetically abnormal cells excessively expressing a HER-2 protein more clearly by carrying out an antigen-antibody reaction using an antibody specifically binding to HER-2. In the present invention, it is preferable to carry out an antigen-antibody reaction using an antibody which specifically binds to one or more proteins selected from the group consisting of Cytokeratin, CD45, HER-2, EpCAM, and FGFR (fibroblast growth factor receptor).

It is possible to detect and analyze genetically abnormal cells with high sensitivity and high efficiency by the method for analyzing genetically abnormal cells of the present invention as defined in the claims. The analysis of genetically abnormal cells is not limited to only a determination of whether cells are genetically abnormal cells or not, or a determination of the degree of gene abnormality, and it is possible to carry out various analyses using an immunostaining method or FISH. For example, with the recent progress of fluorescence image analyzing techniques, it is possible to carry out immunostaining against multiple antigens on identical cells, using multiple fluorescent substances different in their fluorescence property and the like for labeling of antibodies. Thus, by carrying out immunostaining against a target antigen of a particular molecularly-targeted therapeutic agent, in addition to immunostaining which discriminate between a cell group containing genetically abnormal cells and a cell group not containing genetically abnormal cells, in the method for analyzing genetically abnormal cells of the present invention, it is also possible to carry out analysis of expression of the target antigen in the genetically abnormal cells and the like.

### EXAMPLES

The present invention will be described in more detail below by way of examples, but the invention is not limited thereto.

### Example 1

Cells in blood were subjected to immunostaining against CD45 as well as FISH using a Her-2 FISH probe (nucleic acid probe specifically binding to the Her-2 gene) and a CEP17 FISH probe (nucleic acid probe specifically binding to CEP17).

### <Preparation of Cell Sample>

8 mL of blood derived from a stomach cancer patient (BD Vacutainer CPT mononuclear cell-separating blood collection tube, product code: #362753, manufactured by Becton, Dickinson and Company) was subjected to a specific gravity centrifugation treatment at 2,500 rpm for 30 minutes to divide into a plasma fraction and a mononuclear cell layer and a red blood cell layer, and the plasma fraction was removed to recover the mononuclear cell layer. EDTA-containing PBS was added to the recovered mononuclear cell layer to make a volume of 15 mL, and a centrifugation treatment was then carried out at 2,000 rpm for 10 minutes. The precipitated cells were then transferred to a dolphin tube, and further centrifugation treatment (swing arm, manufactured by Eppendorf Co. Ltd.) was carried out at 2,500 rpm for 3 minutes. After removing the supernatant, a MACS (registered trademark) buffer (manufactured by Miltenyi Boitec K. K.) was added to the precipitated cells to obtain 60 µL of a cell suspension.

20 µL of CD45 microbeads (CD45-MB, manufactured by Miltenyi Boitec K. K.) and 20 µL of CD61 microbeads (CD61-MB, manufactured by Miltenyi Boitec K. K.) were added to the cell suspension thus prepared, and the mixture was incubated at 4°C for 15 minutes. Non-binding microbeads in the cell suspension were washed with 1 mL of a MACS (registered trademark) buffer, and cells labeled with the microbeads were then suspended in 500 µL of a MACS (registered trademark) buffer. Subsequently, a dolphin tube containing the suspension was mounted to AutoMACS (registered trademark, manufactured by Miltenyi Boitec K. K.), a "Depletes" program was conducted, and an N1 fraction (cell group binding to neither CD45-MB nor CD61-MB) was recovered in two dolphin tubes. The two dolphin tubes were subjected to a centrifugation treatment at 2,000 rpm for 10 minutes, and precipitated cells were then collected in one tube. A centrifugation treatment was again carried out at 2,000 rpm for 10 minutes, and the supernatant was removed to obtain a cell sample.

### <Immunostaining against Cell Surface Antigen and Surfactant Treatment>

50 µL of a diluted solution of CD45-biotin was added to the cell sample thus prepared, and the mixture was incubated at room temperature for 10 minutes. The diluted solution of CD45-biotin was a solution obtained by diluting CD45-biotin (manufactured by Becton, Dickinson and Company) with a MACS (registered trademark) buffer to one tenth. Subsequently, the cells were washed with 1.5 mL of a MACS (registered trademark) buffer, 50 µL of a diluted solution of Streptavidin-Qdot (registered trademark) 605 (SA-Q605) was added to the cells, and the mixture was incubated at room temperature for 15 minutes. Subsequently, the cells were washed with 1.5 mL of a MACS (registered trademark) buffer.

### <Crosslink Treatment>

Next, 5 µL of a 50 mM BS3 solution was added to the cell suspension, and the mixture was immediately pipetted and then incubated at room temperature for 15 minutes. Subsequently, 1 µL of a 1 M Tris buffer was further added to the mixture, which was incubated at room temperature for 10 minutes. Subsequently, the cells were washed twice with 1.5 mL of EDTA-containing PBS.

### <Immobilization Treatment>

The cells after washing were suspended in 50 µL of IS-A [Dako Instruction Reagent A (formalin-containing fixative), manufactured by Dako] to incubate at room temperature for 15 minutes. Subsequently, the cells were washed with 1.5 mL of a MACS (registered trademark) buffer.

### <Immunostaining against Intracellular Antigen and Surfactant Treatment>

A diluted solution of Cytokeratin-AF647 was added to the cell suspension after washing, and the mixture was incubated at room temperature for 15 minutes. The diluted solution of Cytokeratin-AF647 was a solution obtained by diluting Cytokeratin-Alexa 647 (manufactured by Abcam, an antibody obtained by covalently binding Alexa fluor 647 to clone AE1/AE3, 280 µg/mL) with IS-B to one twenty-fifth. The cells after staining were washed with 1.5 mL of a MACS (registered trademark) buffer and then with 1 mL of PBS. Subsequently, the cells were suspended in 50 µL of a potassium chloride solution (0.075 M) to incubate at room temperature for 15 minutes. The suspension was then subjected to a centrifugation treatment, and the supernatant was removed to leave 20 µL of the potassium chloride solution. The resulting cell suspension was dropped on 5 MAS coat slides (manufactured by Matsunami Glass Ind., Ltd.) in an amount of one spot per slide, and air-dried.

### <Proteolytic Enzyme Treatment>

A pepsin solution was added to MAS slides on which cells were mounted, and incubation was carried out at room temperature for 2 minutes. Then, the pepsin solution was removed from the slides, PBS was added to the slides, incubation was carried out at room temperature for 3 minutes, and the slides were air-dried. The pepsin solution was a solution obtained by diluting pepsin (manufactured by SIGMA) with a 10 mM hydrochloric acid solution to adjust to 100 µg/mL.

### <Ethanol Treatment>

MAS slides after the proteolytic enzyme treatment were immersed in a 70% ethanol solution for 1 minute, then in a 85% ethanol solution for 1 minute, further in a 100% ethanol solution for 1 minute, and then air-dried. Subsequently, the slides were further dried by a drier for 5 minutes on a block heater at 45°C.

### <FISH>

Stock solutions of a Her-2 FISH probe (Her-2-SpecrtrumOrange, manufactured by Vysis) and a CEP17 FISH probe (CEP17-SpectrumGreen, manufactured by Vysis) were added to the MAS slides after drying in an amount of 2 µL per spot, cover glasses were further mounted on the slides and sealed with a paper bond. The MAS slides were incubated at 85°C for 3 minutes, and then at 37°C for 18 hours. After incubation, the bond was peeled off, the MAS slides were immersed in a post-hybridization washing solution (2 × SSC, 0.3% NP40) and incubated at 75°C for 5 minutes. The MAS slides were rinsed with 2 × SSC, and excess water was then removed.

### <Nuclear Staining>

To the MAS slides, 2 µL per spot of a DAPI solution was added, and the slides were then sealed with a top coat.

### <Measurement of Fluorescence Signals>

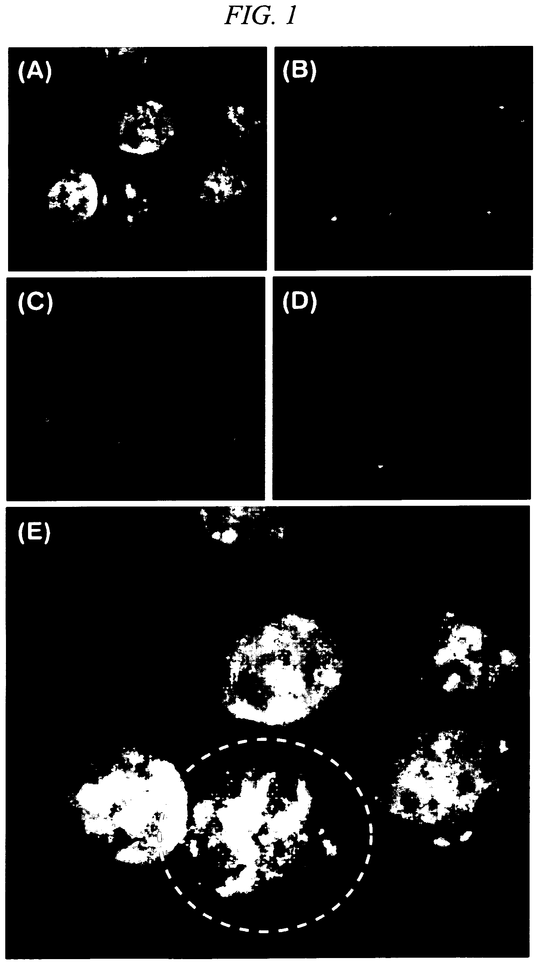

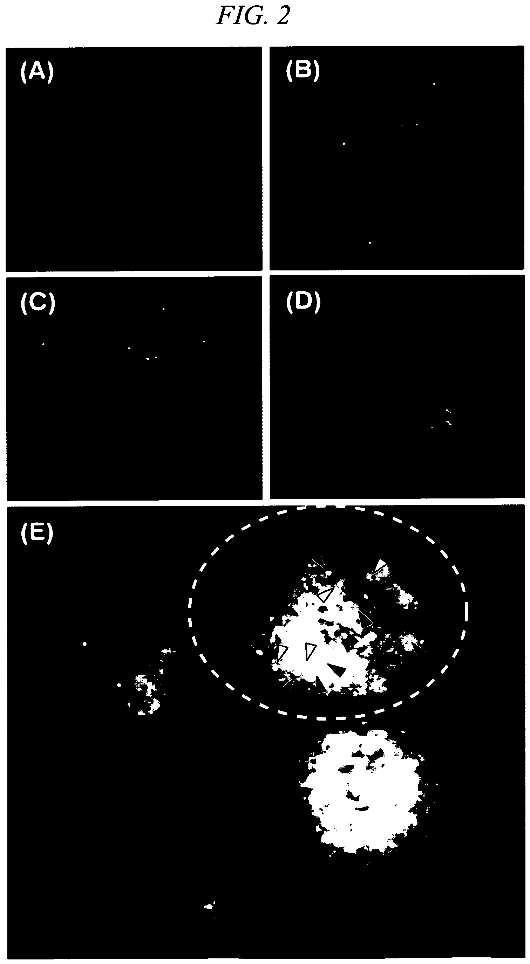

Fluorescence signals in the spots on the MAS slides after sealing were measured using a confocal laser scanning microscope FV-1000 (manufactured by Olympus Corp.) to construct three-dimensional images. Figs. 1 and 2 show two-dimensional images prepared from three-dimensional images reconstructed with respect to one visual field containing Cytokeratin-positive cells, respectively. In Figs. 1 and 2, (A) is a nucleus (DAPI) stained image, (B) is a signal image of the CEP17 FISH probe, (C) is a signal image of the Her-2 FISH probe, (D) is a stained image of Cytokeratin-Alexa 647, and (E) is an image obtained by overlapping (A) to (D).

As a result, signal counts of CEP17 were equal to signal counts of Her-2 in a Cytokeratin-positive cell in Fig. 1 [a cell surrounded by a dotted line in Fig. 1 (E)], and therefore, it was determined that the Her-2 gene of the Cytokeratin-positive cell was normal. On the other hand, in a right upper cell [a cell surrounded by a dotted line in Fig. 2(E)] among Cytokeratin-positive cells (cells stained by Cytokeratin-Alexa 647) in Fig. 2, the total count of CEP17 signals [signals shown by white arrowheads in Fig. 2(E)] was 4, the total count of Her-2 signals [signal shown by black arrowheads in Fig. 2(E)] was 8, and the Her-2/CEP17 ratio was 2. Thus, it was confirmed that a cell surrounded by a dotted line in Fig. 2(E) was a genetically abnormal cell having the Her-2 gene amplified. As is apparent from these results, the method for analyzing genetically abnormal cells of the present invention can analyze Her-2 gene amplification in a portion of Cytokeratin-positive cells contained in blood derived from cancer patients, i.e., that the method for analyzing genetically abnormal cells of the present invention can analyze CTCs in blood derived from cancer patients.

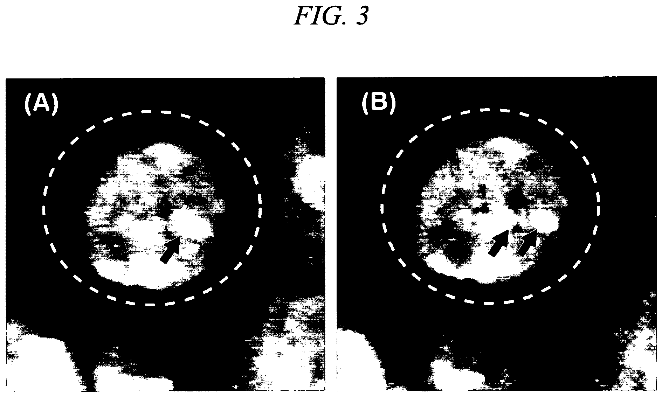

Fig. 3 shows two-dimensional images prepared from three-dimensional images reconstructed with respect to certain cells in which fluorescence signals were observed in this Example. Figs. 3 (A) and 3(B) show two-dimensional images slightly different in an observing point. Figs. 3 (A) and 3(B) are images obtained by overlapping a nucleus (DAPI) stained image and a signal image of a Her-2 FISH probe, and signals of the Her-2 FISH probe are shown by arrows. Signals of the Her-2 FISH probe in cells surrounded by a dotted line in Figs. 3 (A) and 3(B) were detected as one signal in the two-dimensional image in Fig. 3(A) but detected as two separate signals in the two-dimensional image in Fig. 3(B). Thus, in the method for analyzing genetically abnormal cells of the present invention, multiple signals which are detected as one signal due to overlap in a Z-axis direction in a particular two-dimensional image can be detected easily in an individually separated manner by rotating a reconstructed three-dimensional image slightly.

### Example 2

According to the method for analyzing genetically abnormal cells of the present invention, immunostaining against CD45 as well as FISH using a Her-2 FISH probe and a CEP17 FISH probe were carried out on endoscopic biopsy cells.

### <Preparation of Cell Sample>

An endoscopic biopsy (about 1 mg) obtained from a stomach cancer patient was suspended in 0.25 mL of a DMEM medium, collagenase and dispase were then added to the suspension, and an enzyme treatment was carried out at 37°C for 60 minutes. Subsequently, the suspension was subjected to a centrifugation treatment at 2,000 rpm for 10 minutes, precipitated cells (mononuclear cells) were transferred to a dolphin tube, and a further centrifugation treatment (swing arm, manufactured by Eppendorf Co. Ltd.) was carried out at 2,500 rpm for 3 minutes. After removing the supernatant, a MACS (registered trademark) buffer (manufactured by Miltenyi Boitec K. K.) was added to the precipitated cells to obtain 60 µL of a cell suspension.

A cell sample was prepared from the cell suspension prepared, in the same manner as in Example 1, by removing blood cell components by magnetic separation using CD45 microbeads and CD235a microbeads.

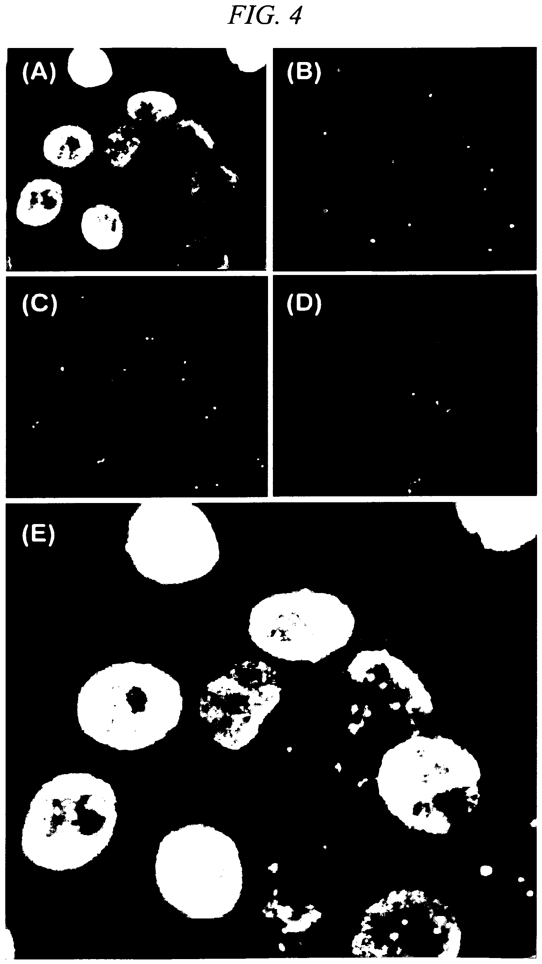

The cell sample prepared was sequentially subjected to immunostaining against a cell surface antigen and a surfactant treatment, a crosslink treatment, an immobilization treatment, immunostaining using Cytokeratin-AF647 and a surfactant treatment, a proteolytic enzyme treatment, an ethanol treatment, FISH, and nuclear staining in the same manner as in Example 1. Subsequently, fluorescence signals of stained cells were measured to construct three-dimensional images. Fig. 4 shows two-dimensional images prepared from the three-dimensional images reconstructed with respect to one visual field containing Cytokeratin-positive cells. In Fig. 4, Fig. 4(A) is a nucleus (DAPI) stained image, Fig. 4(B) is a signal image of the CEP17 FISH probe, Fig. 4(C) is a signal image of the Her-2 FISH probe, Fig. 4(D) is a stained image of Cytokeratin-Alexa 647, Fig. 4(E) is an image obtained by overlapping Fig. 4(A) to 4(D). As a result, signal counts of CEP17 were almost equal to signal counts of Her-2 in Cytokeratin-positive cells in Fig. 4, and therefore, it was determined that the Her-2 gene of the Cytokeratin-positive cells was normal.

### Example 3

A cell sample was prepared by adding a tumor cell line to peripheral blood, and Her-2 gene-amplifying cells in the cell sample were analyzed according to the method for analyzing genetically abnormal cells of the present invention. As a tumor cell line, SKBr3 cells were used which were a cultured cell line derived from breast cancer. In this case, SKBr3 cells cultured by a conventional method were used.

### <Preparation of Cell Sample>

First, 5,000 SKBr3 cells were added to 7.5 mL of peripheral blood obtained from healthy subjects. A mononuclear cell layer was recovered from the peripheral blood and further washed, and a MACS (registered trademark) buffer was then added to the layer to prepare 60 µL of a cell suspension, in the same manner as in Example 1.

To the cell suspension thus prepared, 20 µL of CD45 microbeads (CD45-MB, manufactured by Miltenyi Boitec K. K.) and 20 µL of CD235a microbeads (CD235a-MB, manufactured by Miltenyi Boitec K. K.) were added, and the mixture was incubated at 4°C for 15 minutes. Non-binding microbeads in the cell suspension were washed with 1 mL of a MACS (registered trademark) buffer, and cells labeled with the microbeads were then suspended in 500 µL of a MACS (registered trademark) buffer. Subsequently, a dolphin tube containing the suspension was loaded on AutoMACS (registered trademark, manufactured by Miltenyi Boitec K. K.) "Depletes", a "Depletes" program was conducted, and an N1 fraction (cell group binding to neither CD45-MB nor CD235a-MB) was recovered in two dolphin tubes. The two dolphin tubes were subjected to a centrifugation treatment at 2,000 rpm for 10 minutes, and precipitated cells were then collected in one tube. A centrifugation treatment was again carried out at 2,000 rpm for 10 minutes, and the supernatant was removed to obtain a cell sample.

### <Immunostaining against Cell Surface Antigen>

50 µL of the diluted solution of CD45-biotin used in Example 1 was added to the cell sample prepared, and the mixture was incubated at room temperature for 10 minutes. The cells were washed with 1.5 mL of a MACS (registered trademark) buffer, 50 µL of a diluted solution of Streptavidin-Qdot (registered trademark) 605 (SA-Q605) was added to the cells, and the mixture was incubated at room temperature for 15 minutes. In this connection, the diluted solution of SA-Q605 was a solution obtained by diluting SA-Q605 (manufactured by Invitrogen) with a MACS (registered trademark) buffer to one hundredth. Subsequently, the cells were washed twice with 1.5 mL of EDTA-containing PBS.

### <Immobilization Treatment>

The cells after a permeation treatment were suspended in 50 µL of IS-A [Dako Instruction ReagentA (formalin-containing fixative), manufactured by Dako] to incubate at room temperature for 15 minutes. Subsequently, the cells were washed with 1.5 mL of a MACS (registered trademark) buffer.

### <Immunostaining against Intracellular Antigen>

To the cell suspension after washing, the diluted solution of Cytokeratin-AF647 used in Example 1 was added, and the mixture was incubated at room temperature for 15 minutes. The cells after staining were washed with 1.5 mL of a MACS (registered trademark) buffer and then with 1 mL of PBS. Subsequently, the cells were suspended in 50 µL of a potassium chloride solution (0.075 M) to incubate at room temperature for 15 minutes. The suspension was then subjected to a centrifugation treatment, and the supernatant was removed to leave 20 µL of the potassium chloride solution. The resulting cell suspension was dropped on 5 MAS coat slides (manufactured by Matsunami Glass Ind., Ltd.) in an amount of one spot per slide, and air-dried.

### <Proteolytic Enzyme Treatment>

The pepsin solution used in Example 1 was added to MAS slides on which cells were mounted, and incubation was carried out at room temperature for 2 minutes. Then, the pepsin solution was removed from the slides, PBS was added to the slides, incubation was carried out at room temperature for 3 minutes, and the slides were air-dried.

### <Ethanol Treatment>

MAS slides after the proteolytic enzyme treatment were immersed in a 70% ethanol solution for 1 minute, then in an 85% ethanol solution for 1 minute, further in a 100% ethanol solution for 1 minute, and then air-dried. Subsequently, the slides were further dried by applying a drier for 5 minutes on a block heater at 45°C.

### <FISH and Nuclear Staining>

FISH was carried out on MAS slides after drying, in the same manner as in Example 1. Subsequently, a DAPI solution was added to the slides, and the slides were sealed with a top coat.

### <Measurement of Fluorescence Signals>

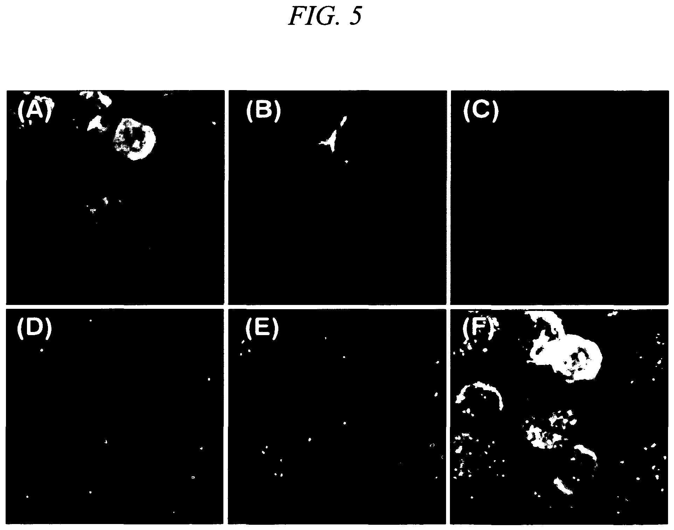

Fluorescence signals in the spots on the MAS slides after sealing were measured using confocal laser scanning microscope FV-1000 (manufactured by Olympus Corp.) to construct three-dimensional images. Fig. 5 shows two-dimensional images prepared from three-dimensional images reconstructed with respect to one visual field containing Cytokeratin-positive cells. In Fig. 5, Fig. 5(A) is a nucleus (DAPI) stained image, Fig. 5(B) is a stained image of CD45-biotin/Streptavidin-Qdot, Fig. 5(C) is a stained image of Cytokeratin-Alexa 647, Fig. 5(D) is a signal image of the CEP17 FISH probe, Fig. 5(E) is a signal image of the Her-2 FISH probe, and Fig. 5(F) is an image obtained by overlapping Figs. 5(A) to 5(E).

As a result, the proportion of Cytokeratin-positive cells in total cells contained in the cell sample was very high, and the Cytokeratin-positive cells were efficiently concentrated by a magnetic separation treatment using CD45 microbeads and CD235a microbeads, as is apparent from Figs. 5 (B) and 5(C). Also, the ratio of signal counts of CEP17 to signal counts of Her-2 in Cytokeratin-positive/CD45-negative cells in Fig. 5 was 3.5 on average, and therefore, it was determined that the Her-2 gene of the Cytokeratin-positive cells was amplified.

### Reference Example 1

A cell sample was prepared for analyzing CTCs in blood, and epithelial cells contained in the cell sample were detected by immunostaining, in the same manner as in Example 1.

First, a mononuclear cell layer was recovered from 8 mL of blood derived from a stomach cancer patient (BD Vacutainer CPT mononuclear cell-separating blood collection tube, product code: #362753, manufactured by Becton, Dickinson and Company) and further washed, and a MACS (registered trademark) buffer was then added to the layer to prepare 60 µL of a cell suspension, in the same manner as in Example 1. A cell sample was prepared from the cell suspension thus prepared, in the same manner as in Example 1, by removing leukocyte components by magnetic separation using CD45 microbeads and CD61 microbeads.

Next, 25 µL of a diluted solution of EpCAM-Alexa Fluor 488 and 25 µL of a diluted solution of CD45-biotin were added to the cell sample thus prepared, and the mixture was incubated at room temperature for 10 minutes. The diluted solution of EpCAM-Alexa Fluor 488 was a solution obtained by diluting EpCAM-Alexa Fluor 488 (manufactured by BioLegend) with a MACS (registered trademark) buffer to one twentieth. The diluted solution of CD45-biotin was a solution obtained by diluting CD45-biotin (manufactured by Becton, Dickinson and Company) with a MACS (registered trademark) buffer to one fifth. Subsequently, the cells were washed with 1.5 mL of a MACS (registered trademark) buffer, 50 µL of a diluted solution of Streptavidin-Qdot (registered trademark) 605 (SA-Q605) was added to the cells, and the mixture was incubated at room temperature for 15 minutes. Subsequently, the cells were washed with 1.5 mL of a MACS (registered trademark) buffer.

The cells after a permeation treatment were sequentially subjected to an immobilization treatment, immunostaining using Cytokeratin-AF647, a proteolytic enzyme treatment, and an ethanol treatment in the same manner as in Example 3. Subsequently, in order to carry out nuclear staining, 2 µL per spot of a DAPI solution was added to MAS slides after drying, and the slides were then sealed with a top coat.

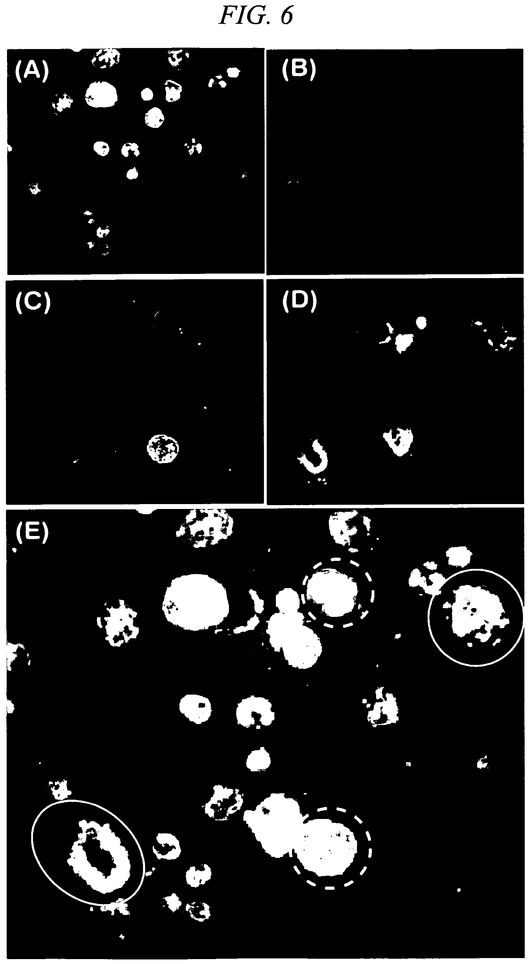

Fluorescence signals in the spots on the MAS slides after sealing were measured using confocal laser scanning microscope FV-1000 (manufactured by Olympus Corp.). Fig. 6 shows two-dimensional images prepared from three-dimensional images reconstructed with respect to one visual field containing Cytokeratin-positive cells. In Fig. 6, Fig. 6(A) is a nucleus (DAPI) stained image, Fig. 6(B) is a stained image of CD45-biotin/Streptavidin-Qdot, Fig. 6(C) is a stained image of EpCAM-Alexa Fluor 488, Fig. 6(D) is a stained image of Cytokeratin-Alexa 647, and Fig. 6(E) is an image obtained by overlapping (A) to (D).

As is apparent from Fig. 6(C) and Fig. 6(D), expression of an EpCAM antigen remarkably differs from one Cytokeratin-positive cell to another, and the cells contain both EpCAM-positive cells and EpCAM-negative cells. For example, cells in the region surrounded by a dashed line in Fig. 6(E) are Cytokeratin-positive and EpCAM-positive. On the other hand, cells in the region surrounded by a solid line are Cytokeratin-positive but express little EpCAM. Thus, in case EpCAM-positive cells are selectively recovered from a mononuclear cell layer recovered from blood as in the method described in Flores et al., British Journal of Cancer, 2010, Vol. 102, No. 10, p.1495-1502., EpCAM-negative CTCs are excluded from the analysis object, and therefore, it is difficult to detect CTCs in a blood specimen accurately. Accordingly, in order to increase the proportion of the presence of CTCs in a cell sample when analyzing CTCs in a blood specimen, the method for analyzing genetically abnormal cells of the present invention does not adopt a step of selectively recovering epithelial cells from a mononuclear cell layer recovered from blood utilizing a cell surface antigen on epithelial cells, but preferably adopts a step of separating and removing blood cells utilizing a cell surface antigen on blood cells.

### INDUSTRIAL APPLICABILITY

According to the method for analyzing genetically abnormal cells of the present invention, since it is possible to detect and analyze genetically abnormal cells, which exist in a normal cell group only in a trace amount, in a highly accurate and simple manner, the present method is suitably utilized in the fields of clinical tests such as analysis of peripheral circulatory tumor cells and the like.

## Claims

1. A method of analyzing genetically abnormal cells, the method comprising the steps of:
(a) preparing a cell sample containing eukaryotic cells, wherein the cell sample is blood or a mononuclear cell component separated from blood,
(b) immunostaining of cells contained in the cell sample using one or more antibodies against a cell surface antigen of genetically abnormal cells after the step (a),
(c) subjecting the cells contained in the cell sample to a permeation treatment after the step (b),
(d) subjecting the cells contained in the cell sample to an immobilization treatment after the step (b),
(e) conducting FISH (fluorescence in situ hybridization) to the cells contained in the cell sample using one or more nucleic acid probes specifically binding to DNA of a cancer gene after the step (d),
(f) analyzing fluorescence signals from the nucleic acid probes in the cells contained in the cell sample using a three-dimensional image analysis method after the step (e), and
(g) determining whether the cells contained in the cell sample are genetically abnormal cells or not based on a results of the step (b) and a results of the step (f) by analyzing results of the FISH on cells having bound the antibody of the step (b), further comprising the step of
(i) immunostaining of cells contained in the cell sample using one or more antibodies which specifically bind to a molecule existing in the interior of cells likely to be genetically abnormal before the step (g) after the step (c).

2. A method of analyzing genetically abnormal cells, the method comprising the steps of:
(a) preparing a cell sample containing eukaryotic cells, wherein the cell sample is blood or a mononuclear cell component separated from blood,
(b) immunostaining of cells contained in the cell sample using one or more antibodies against a cell surface antigen of non-genetically abnormal cells after the step (a),
(c) subjecting the cells contained in the cell sample to a permeation treatment after the step (b),
(d) subjecting the cells contained in the cell sample to an immobilization treatment after the step (b),
(e) conducting FISH (fluorescence in situ hybridization) to the cells contained in the cell sample using one or more nucleic acid probes specifically binding to DNA of a cancer gene after the step (d),
(f) analyzing fluorescence signals from the nucleic acid probes in the cells contained in the cell sample using a three-dimensional image analysis method after the step (e), and
(g) determining whether the cells contained in the cell sample are genetically abnormal cells or not based on a results of the step (b) and a results of the step (f) by analyzing results of the FISH on cells having not bound the antibody of the step (b), further comprising the step of
(i) immunostaining of cells contained in the cell sample using one or more antibodies which specifically bind to a molecule existing in the interior of cells likely to be genetically abnormal before the step (g) after the step (c).

3. The method of analyzing genetically abnormal cells according to claim 1 or 2, wherein the permeation treatment is carried out by an enzyme reaction treatment with a proteolytic enzyme after an immersion treatment in a surfactant solution.

4. The method of analyzing genetically abnormal cells according to claim 1, further comprising the step of:
(h) removing cells binding to the antibody from the cell sample prepared in the step (a) using an antibody which specifically binds to a molecule existing on a cell surface of other cells than genetically abnormal cells which are analysis objects before the step (b), wherein
the step (b) is the step of subjecting cells remaining after removal of a portion of cells by the step (h) to an antigen-antibody reaction.

5. The method of analyzing genetically abnormal cells according to claim 1, wherein the antibody used in the step (b) is an antibody which specifically binds a molecule mainly existing on the cell surface of epithelial cells.

6. The method of analyzing genetically abnormal cells according to claim 5, wherein the antibody specifically binds to one or more proteins selected from the group consisting of Ep-CAM and HER-2; preferably wherein the antibody specifically binds to EpCAM.

7. The method of analyzing genetically abnormal cells according to claim 5, wherein the antibody specifically binds to EpCAM.

8. The method of analyzing genetically abnormal cells according to claim 2, wherein the antibody used in the step (b) is an antibody which specifically binds to leukocytes, platelets, red blood cells, or endothelial cells.

9. The method of analyzing genetically abnormal cells according to claim 8, wherein the antibody specifically binds to CD45, CD61, CD235a, or CD31.

10. The method of analyzing genetically abnormal cells according to claim 8, wherein antibody used in the step (b) is an antibody which specifically binds to CD45.

11. The method of analyzing genetically abnormal cells according to any one of claims 1 to 10, wherein the antibody used in the step (i) is an antibody which specifically binds to Cytokeratin.

12. The method of analyzing genetically abnormal cells according to any one of claims 1 to 11, wherein
the nucleic acid probe is a nucleic acid probe specifically binding to DNA of Her-2 gene, and a nucleic acid probe specifically binding to DNA of CEP17 gene.

13. The method of analyzing genetically abnormal cells according to claim 4, wherein
the antibody used in the step (h) is an antibody which specifically binds to a molecule specifically existing on a cell surface of leukocyte cells; and
the nucleic acid probe is a nucleic acid probe specifically binding to DNA of the Her-2 gene, and a nucleic acid probe specifically binding to DNA of the CEP17 gene.

14. The method of analyzing genetically abnormal cells according to claim 4, wherein
the antibody used in the step (b) is an antibody which specifically binds to a molecule specifically existing on a cell surface of leukocyte cells;
the antibody used in the step (h) is an antibody which specifically binds to a molecule specifically existing on a cell surface of leukocyte cells;
the antibody used in the step (i) is an antibody which specifically binds to Cytokeratin; and
the nucleic acid probe is a nucleic acid probe specifically binding to DNA of Her-2 gene, and a nucleic acid probe specifically binding to DNA of CEP17 gene.

15. The method of analyzing genetically abnormal cells according to claim 3, wherein
the surfactant solution is triton X-100; and
the proteolytic enzyme is pepsin.

## Patentansprüche

1. Verfahren zum Analysieren von genetisch abnormalen Zellen, wobei das Verfahren die Schritte umfasst:
(a) Herstellen einer eukaryotische Zellen enthaltenden Zellprobe, wobei die Zellprobe Blut oder ein aus Blut abgetrennter mononuklearer Zellbestandteil ist,
(b) Immunfärben der in der Zellprobe enthaltenen Zellen unter Verwendung von einem oder mehreren Antikörpern gegen ein Zelloberflächenantigen von genetisch abnormalen Zellen nach dem Schritt (a),
(c) Unterziehen der in der Zellprobe enthaltenden Zellen einer Permeationsbehandlung nach dem Schritt (b),
(d) Unterziehen der in der Zellprobe enthaltenden Zellen einer Immobilisierungsbehandlung nach dem Schritt (b),
(e) Durchführen von FISH (Fluoreszenz in situ Hybridisierung) gegenüber der in der Zellprobe enthaltenen Zellen unter Verwendung von einer oder mehreren Nukleinsäuresonden, die spezifisch an DNA eines Krebsgens binden nach dem Schritt (d),
(f) Analysieren von Fluoreszenzsignalen von den Nukleinsäuresonden in den in der Zellprobe enthaltenen Zellen unter Verwendung eines dreidimensionalen Bildanalyseverfahren nach dem Schritt (e), und
(g) Bestimmen, ob die in der Zellprobe enthaltenen Zellen genetisch abnormale Zellen sind oder nicht, basierend auf einem Ergebnis des Schritt (b) und einem Ergebnis des Schritt (f) durch Analysieren von Ergebnissen der FISH in Zellen, die den Antikörper des Schritts (b) gebunden haben, des Weiteren umfassend den Schritt des
(i) Immunfärbens der in der Zellprobe enthaltenen Zellen unter Verwendung eines oder mehrerer Antikörper, die spezifisch an ein Molekül binden, das in dem Inneren von Zellen existiert, für die es wahrscheinlich ist, genetisch abnormal zu sein, vor dem Schritt (g) nach dem Schritt (c).

2. Verfahren zum Analysieren von genetisch abnormalen Zellen, wobei das Verfahren die Schritte umfasst:
(a) Herstellen einer eukaryotische Zellen enthaltenden Zellprobe, wobei die Zellprobe Blut oder ein aus Blut abgetrennter mononuklearer Zellbestandteil ist,
(b) Immunfärben der in der Zellprobe enthaltenen Zellen unter Verwendung von einem oder mehreren Antikörpern gegen ein Zelloberflächenantigen von nicht-genetisch abnormalen Zellen nach dem Schritt (a),
(c) Unterziehen der in der Zellprobe enthaltenden Zellen einer Permeationsbehandlung nach dem Schritt (b),
(d) Unterziehen der in der Zellprobe enthaltenden Zellen einer Immobilisierungsbehandlung nach dem Schritt (b),
(e) Durchführen von FISH (Fluoreszenz in situ Hybridisierung) gegenüber der in der Zellprobe enthaltenen Zellen unter Verwendung von einer oder mehreren Nukleinsäuresonden, die spezifisch an DNA eines Krebsgens binden nach dem Schritt (d),
(f) Analysieren von Fluoreszenzsignalen von den Nukleinsäuresonden in den in der Zellprobe enthaltenen Zellen unter Verwendung eines dreidimensionalen Bildanalyseverfahren nach dem Schritt (e), und
(g) Bestimmen, ob die in der Zellprobe enthaltenen Zellen genetisch abnormale Zellen sind oder nicht, basierend auf einem Ergebnis des Schritt (b) und einem Ergebnis des Schritt (f) durch Analysieren von Ergebnissen der FISH in Zellen, die den Antikörper des Schritts (b) nicht gebunden haben, des Weiteren umfassend den Schritt des
(i) Immunfärbens der in der Zellprobe enthaltenen Zellen unter Verwendung eines oder mehrerer Antikörper, die spezifisch an ein Molekül binden, das in dem Inneren von Zellen existiert, für die es wahrscheinlich ist, genetisch abnormal zu sein, vor dem Schritt (g) nach dem Schritt (c).

3. Verfahren des Analysierens von genetisch abnormalen Zellen nach Anspruch 1 oder 2, wobei die Permeationsbehandlung durchgeführt wird durch eine Enzymreaktionsbehandlung mit einem proteolytischen Enzym nach einer Immersionsbehandlung in einer Lösung eines oberflächenaktiven Mittels.

4. Verfahren des Analysierens von genetisch abnormalen Zellen nach Anspruch 1, des Weiteren umfassend den Schritt:
(h) Entfernen von den Antikörper bindenden Zellen aus der in Schritt (a) hergestellten Zellprobe unter Verwendung eines Antikörpers, der spezifisch an ein Molekül bindet, das auf einer Zelloberfläche von anderen Zellen als genetisch abnormalen Zellen existiert, die Analyseobjekte sind vor dem Schritt (b), wobei
der Schritt (b) der Schritt des Unterziehens der nach der Entfernung eines Teils der Zellen durch Schritt (h) verbleibenden Zellen einer Antigen-Antikörper-Reaktion ist.

5. Verfahren des Analysierens von genetisch abnormalen Zellen nach Anspruch 1, wobei der in dem Schritt (b) verwendete Antikörper ein Antikörper ist, der spezifisch ein Molekül bindet, das hauptsächlich auf der Zelloberfläche von Epithelzellen existiert.

6. Verfahren des Analysierens von genetisch abnormalen Zellen nach Anspruch 5, wobei der Antikörper spezifisch an ein oder mehrere Proteine bindet, ausgewählt aus der Gruppe bestehend aus Ep-CAM und HER-2; vorzugsweise wobei der Antikörper spezifisch an EpCAM bindet.

7. Verfahren des Analysierens von genetisch abnormalen Zellen nach Anspruch 5, wobei der Antikörper spezifisch an EpCAM bindet.

8. Verfahren des Analysierens von genetisch abnormalen Zellen nach Anspruch 2, wobei der in Schritt (b) verwendete Antikörper ein Antikörper ist, der spezifisch Leukozyten, Plättchen, rote Blutzellen oder Endothelzellen bindet.

9. Verfahren des Analysierens von genetisch abnormalen Zellen nach Anspruch 8, wobei der Antikörper spezifisch an CD45, CD61, CD235a oder CD31 bindet.

10. Verfahren des Analysierens von genetisch abnormalen Zellen nach Anspruch 8, wobei der in Schritt (b) verwendete Antikörper ein Antikörper ist, der spezifisch an CD45 bindet.

11. Verfahren des Analysierens von genetisch abnormalen Zellen nach einem beliebigen der Ansprüche 1 bis 10, wobei der in Schritt (i) verwendete Antikörper ein Antikörper ist, der an Cytokeratin bindet.

12. Verfahren des Analysierens von genetisch abnormalen Zellen nach einem beliebigen der Ansprüche 1 bis 11, wobei die Nukleinsäuresonde eine Nukleinsäuresonde ist, die spezifisch an DNA des Her-2 Gens bindet, und eine Nukleinsäure, die spezifisch an DNA des CEP17 Gens bindet.

13. Verfahren des Analysierens von genetisch abnormalen Zellen nach Anspruch 4, wobei der in Schritt (h) verwendete Antikörper ein Antikörper ist, der spezifisch an ein Molekül bindet, das spezifisch auf einer Zelloberfläche von Leukozytenzellen existiert; und
die Nukleinsäuresonde eine Nukleinsäuresonde ist, die spezifisch an DNA des Her-2 Gens bindet, und eine Nukleinsäuresonde, die spezifisch an DNA des CEP17 Gens bindet.

14. Verfahren des Analysierens von genetisch abnormalen Zellen nach Anspruch 4, wobei
der in Schritt (b) verwendete Antikörper ein Antikörper ist, der spezifisch an ein Molekül bindet, das spezifisch auf einer Zelloberfläche von Leukozytenzellen existiert;
der in Schritt (h) verwendete Antikörper ein Antikörper ist, der spezifisch an ein Molekül bindet, das spezifisch auf einer Zelloberfläche von Leukozytenzellen existiert;
der in Schritt (i) verwendete Antikörper ein Antikörper ist, der spezifisch an Cytokeratin bindet; und
die Nukleinsäuresonde eine Nukleinsäuresonde ist, die spezifisch an DNA des Her-2 Gens bindet, und eine Nukleinsäuresonde, die spezifisch an DNA des CEP17 Gens bindet.

15. Verfahren des Analysierens von genetisch abnormalen Zellen nach Anspruch 3, wobei
die Lösung des oberflächenaktiven Mittels Triton X-100 ist; und
das proteolytische Enzym Pepsin ist.

## Revendications

1. Procédé d'analyse de cellules génétiquement anormales, le procédé comprenant les étapes de :
(a) préparation d'un échantillon de cellules contenant des cellules eucaryotes, dans lequel l'échantillon de cellules est du sang ou un constituant de cellule mononucléaire séparé du sang,
(b) immunocoloration des cellules contenues dans l'échantillon de cellules au moyen d'un ou de plusieurs anticorps contre un antigène de surface cellulaire de cellules génétiquement anormales après l'étape (a),
(c) soumission des cellules contenues dans l'échantillon de cellules à un traitement de perméation après l'étape (b),
(d) soumission des cellules contenues dans l'échantillon de cellules à un traitement d'immobilisation après l'étape (b),
(e) réalisation d'une FISH (hybridation in situ en fluorescence) sur les cellules contenues dans l'échantillon de cellules au moyen d'une ou de plusieurs sondes d'acide nucléique se liant spécifiquement à l'ADN d'un gène de cancer après l'étape (d),
(f) analyse des signaux de fluorescence provenant des sondes d'acide nucléique dans les cellules contenues dans l'échantillon de cellules au moyen d'une méthode d'analyse d'image tridimensionnelle après l'étape (e), et
(g) détermination si les cellules contenues dans l'échantillon de cellules sont des cellules génétiquement anormales ou non sur la base des résultats de l'étape (b) et des résultats de l'étape (f) en analysant les résultats de la FISH sur les cellules ayant lié l'anticorps de l'étape (b), comprenant en outre l'étape de
(i) immunocoloration des cellules contenues dans l'échantillon de cellules au moyen d'un ou de plusieurs anticorps qui se lient spécifiquement à une molécule existant à l'intérieur des cellules susceptibles d'être génétiquement anormales avant l'étape (g) après l'étape (c).

2. Procédé d'analyse de cellules génétiquement anormales, le procédé comprenant les étapes de :
(a) préparation d'un échantillon de cellules contenant des cellules eucaryotes, dans lequel l'échantillon de cellules est du sang ou un constituant de cellule mononucléaire séparé du sang,
(b) immunocoloration des cellules contenues dans l'échantillon de cellules au moyen d'un ou de plusieurs anticorps contre un antigène de surface cellulaire de cellules non génétiquement anormales après l'étape (a),
(c) soumission des cellules contenues dans l'échantillon de cellules à un traitement de perméation après l'étape (b),
(d) soumission des cellules contenues dans l'échantillon de cellules à un traitement d'immobilisation après l'étape (b),
(e) réalisation d'une FISH (hybridation in situ en fluorescence) sur les cellules contenues dans l'échantillon de cellules au moyen d'une ou de plusieurs sondes d'acide nucléique se liant spécifiquement à l'ADN d'un gène de cancer après l'étape (d),
(f) analyse des signaux de fluorescence provenant des sondes d'acide nucléique dans les cellules contenues dans l'échantillon de cellules au moyen d'une méthode d'analyse d'image tridimensionnelle après l'étape (e), et
(g) détermination si les cellules contenues dans l'échantillon de cellules sont des cellules génétiquement anormales ou non sur la base des résultats de l'étape (b) et des résultats de l'étape (f) en analysant les résultats de la FISH sur les cellules n'ayant pas lié l'anticorps de l'étape (b), comprenant en outre l'étape de
(i) immunocoloration des cellules contenues dans l'échantillon de cellules au moyen d'un ou de plusieurs anticorps qui se lient spécifiquement à une molécule existant à l'intérieur des cellules susceptibles d'être génétiquement anormales avant l'étape (g) après l'étape (c).

3. Procédé d'analyse de cellules génétiquement anormales selon la revendication 1 ou 2, dans lequel le traitement de perméation est réalisé par un traitement de réaction enzymatique avec une enzyme protéolytique après un traitement d'immersion dans une solution de tensioactif.

4. Procédé d'analyse de cellules génétiquement anormales selon la revendication 1, comprenant en outre l'étape de :
(h) élimination des cellules se liant à l'anticorps de l'échantillon de cellules préparé à l'étape (a) au moyen d'un anticorps qui se lie spécifiquement à une molécule existant sur une surface cellulaire d'autres cellules que les cellules génétiquement anormales qui font l'objet de l'analyse avant l'étape (b), dans lequel l'étape (b) est l'étape de soumission des cellules restantes après l'élimination d'une partie des cellules par l'étape (h) à une réaction antigène-anticorps.

5. Procédé d'analyse de cellules génétiquement anormales selon la revendication 1, dans lequel l'anticorps utilisé à l'étape (b) est un anticorps qui se lie spécifiquement à une molécule existant principalement sur la surface cellulaire des cellules épithéliales.

6. Procédé d'analyse de cellules génétiquement anormales selon la revendication 5, dans lequel l'anticorps se lie spécifiquement à une ou plusieurs protéines choisies dans le groupe constitué par Ep-CAM et HER-2 ; de préférence dans lequel l'anticorps se lie spécifiquement à EpCAM.

7. Procédé d'analyse de cellules génétiquement anormales selon la revendication 5, dans lequel l'anticorps se lie spécifiquement à EpCAM.

8. Procédé d'analyse de cellules génétiquement anormales selon la revendication 2, dans lequel l'anticorps utilisé à l'étape (b) est un anticorps qui se lie spécifiquement aux leucocytes, aux plaquettes, aux globules rouges ou aux cellules endothéliales.

9. Procédé d'analyse de cellules génétiquement anormales selon la revendication 8, dans lequel l'anticorps se lie spécifiquement à CD45, CD61, CD235a ou CD31.

10. Procédé d'analyse de cellules génétiquement anormales selon la revendication 8, dans lequel l'anticorps utilisé à l'étape (b) est un anticorps qui se lie spécifiquement à CD45.

11. Procédé d'analyse de cellules génétiquement anormales selon l'une quelconque des revendications 1 à 10, dans lequel l'anticorps utilisé à l'étape (i) est un anticorps qui se lie spécifiquement à la cytokératine.

12. Procédé d'analyse de cellules génétiquement anormales selon l'une quelconque des revendications 1 à 11, dans lequel la sonde d'acide nucléique est une sonde d'acide nucléique se liant spécifiquement à l'ADN du gène Her-2, et une sonde d'acide nucléique se liant spécifiquement à l'ADN du gène CEP17.

13. Procédé d'analyse de cellules génétiquement anormales selon la revendication 4, dans lequel
l'anticorps utilisé à l'étape (h) est un anticorps qui se lie spécifquement à une molécule existant spécifiquement sur une surface cellulaire des cellules leucocytaires ; et
la sonde d'acide nucléique est une sonde d'acide nucléique se liant spécifiquement à l'ADN du gène Her-2, et une sonde d'acide nucléique se liant spécifiquement à l'ADN du gène CEP17.

14. Procédé d'analyse de cellules génétiquement anormales selon la revendication 4, dans lequel
l'anticorps utilisé à l'étape (b) est un anticorps qui se lie spécifiquement à une molécule existant spécifiquement sur une surface cellulaire des cellules leucocytaires ;
l'anticorps utilisé à l'étape (h) est un anticorps qui se lie spécifiquement à une molécule existant spécifiquement sur une surface cellulaire des cellules leucocytaires ;
l'anticorps utilisé à l'étape (i) est un anticorps qui se lie spécifiquement à la cytokératine ; et
la sonde d'acide nucléique est une sonde d'acide nucléique se liant spécifiquement à l'ADN du gène Her-2, et une sonde d'acide nucléique se liant spécifiquement à l'ADN du gène CEP17.

15. Procédé d'analyse de cellules génétiquement anormales selon la revendication 3, dans lequel
la solution de tensioactif est le triton X-100 ; et
l'enzyme protéolytique est la pepsine.
